# EUROPEAN PATENT APPLICATION

(11) **EP 4 559 932 A1**
(43) Date of publication of application: **28.05.2025**
(21) Application number: 23842343.8
(22) Date of filing: 19.07.2023
(51) Int. Cl.: C07K 16/18, C12N 15/13, A61K 39/395, A61P 31/00

(54) **MULTISPECIFIC ANTIBODY AND USE THEREOF**

(30) Priority: 20.07.2022 CN 202210852642
(71) Applicant: Minghui Pharmaceutical (Hangzhou) Limited, Hangzhou, Zhejiang 310018 (CN); Minghui Pharmaceutical (Shanghai) Limited, Pilot Free Trade Zone Pudong New Area Shanghai 201203 (CN)
(72) Inventor: LIU, Bo, Hangzhou, Zhejiang 310018 (CN); ZHU, Liyuan, Shanghai 201203 (CN); CAO, Guoqing, Shanghai 201203 (CN); SHI, Junwei, Shanghai 201203 (CN)
(74) Representative: SONN Patentanwälte GmbH & Co KG
(86) International application number: PCT/CN2023/108073
(87) International publication number: WO 2024/017281

(57) **Abstract**

The present invention relates to a multispecific antibody targeting programmed cell death receptor-1 (PD-1) and a vascular endothelial growth factor (VEGF), a pharmaceutical composition comprising the multispecific antibody, a nucleic acid molecule and a vector encoding the multispecific antibody, a host cell, and use thereof for treating tumors.

## Description

### Cross-reference of related Application

The present application claims priority of Chinese patent application No. 202210852642.9 filed on July 20, 2022, and the entire contents of the patent application are incorporated herein by reference in their entirety.

### Technical Field

The present invention relates to the field of immunology. Specifically, the present invention relates to multispecific antibodies targeting programmed cell death receptor-1 (PD-1) and vascular endothelial growth factor (VEGF), pharmaceutical compositions comprising the multispecific antibodies, nucleic acid molecules encoding the multispecific antibodies, vectors and host cells, and their use in the treatment of tumors.

### Background

Programmed cell death receptor-1 (PD-1) is a type I transmembrane glycoprotein receptor belonging to the CD28 immunoglobulin superfamily. It is widely expressed on immune cells such as T cells and NK cells. PD-1 has two natural ligands, PD-L1 and PD-L2, both of which belong to the B7 superfamily and are constitutively or inducibly expressed on various cell surfaces, including non-hematopoietic cells, immune cells and tumor cells. The interaction between PD-1 and its ligands inhibits activation and proliferation of lymphocytes (such as T cells and NK cells), the secretion of cytokines (e.g., IL-2, IFN-γ) and the killing of tumor cells.

Vascular endothelial growth factor (VEGF) is a highly specific growth factor that promotes endothelial cell proliferation, enhances formation of new blood vessels and increases vascular permeability. The VEGF family includes VEGFA, VEGFB, VEGFC, VEGFD and PIGF. The VEGF receptors (VEGFR) include VEGFR1 (also known as Flt1), VEGFR2 (also known as KDR or Flk1), VEGFR3 (also known as Flt4) and Neuropilin-1 (NRP-1). VEGFR1 and VEGFR2 are primarily located on the surface of vascular endothelial cells, while VEGFR3 is mainly located on lymphatic endothelial cells. The formation of neovascularization provides necessary nutrients and oxygen for the growth, deterioration, and metastasis of tumors, and is an important target for inhibiting tumor growth. Bevacizumab, a humanized monoclonal antibody targeting VEGF-A, has been approved for the treatment of various cancers, including non-small cell lung cancer, renal cell carcinoma, cervical cancer and metastatic colorectal cancer. Ranibizumab, an affinity-enhanced monoclonal antibody Fab fragment specific for VEGF-A, has been approved for the treatment of the "wet" type of age-related macular degeneration, diabetic retinopathy, and macular edema due to branch retinal vein occlusion or central retinal vein occlusion.

Anti-PD-1/PD-L1 monoclonal antibodies combined with VEGF pathway inhibitors (such as Bevacizumab or tyrosine kinase inhibitors like Lenvatinib and Sorafenib) have shown breakthrough efficacy in various tumors, including liver cancer, lung cancer, gastric cancer, kidney cancer and several gynecological cancers. However, the adverse reactions of combination therapy are significantly higher than those observed with monotherapy.

Therefore, there is a need to develop multi-specific antibodies targeting both the PD-1/PD-L1 and VEGF pathways to provide synergistic anti-tumor efficacy while reducing adverse reactions, which also makes clinical treatment more convenient.

### Contents of the Invention

The present invention provides a multispecific antibody targeting both PD-1 and VEGF, capable of specifically blocking the PD-1 and VEGF signaling pathways, thereby exerting a synergistic anti-tumor effect. The application provides the following aspects.

### Multispecific Antibody

In one aspect, the invention provides a multispecific antibody, which comprises a first antigen-binding domain targeting programmed cell death receptor 1 (PD-1) and a second antigen-binding domain targeting vascular endothelial growth factor (VEGF), wherein, the first antigen-binding domain comprises a VHH domain, the VHH domain includes CDR1 as shown in SEQ ID NO: 2 or a variant thereof, CDR2 as shown in SEQ ID NO: 3 or a variant thereof, and CDR3 as shown in SEQ ID NO: 4 or a variant thereof; wherein the variant has a substitution, deletion or addition of one or several amino acids (e.g., substitution, deletion or addition of 1, 2, or 3 amino acids) as compared to the sequence from which it is derived.

In certain embodiments, the VHH domain comprises: CDR1 as shown in SEQ ID NO: 2, CDR2 as shown in SEQ ID NO: 3, and CDR3 as shown in SEQ ID NO: 4.

In certain embodiments, the VHH domain comprises: CDR1, CDR2 and CDR3 as included in the VHH domain as shown in SEQ ID NO: 1; preferably, the CDRs are determined according to the Kabat, IMGT, or Chothia numbering systems.

In certain embodiments, the VHH domain typically consists of four framework regions (FRs) and three complementarity-determining regions (CDRs), referred to as FR1, CDR1, FR2, CDR2, FR3, CDR3, and FR4. The VHH domain may be truncated at the N- terminus or C-terminus to include only portions of FR1 and/or FR4, or lack one or two of these framework regions, provided it substantially retains antigen-binding specificity.

In certain embodiments, the VHH domain may comprise framework region sequences derived from camelid heavy chain antibodies.

In certain embodiments, the VHH domain may be humanized, wherein one or more of the framework regions have been replaced with human framework regions. In certain embodiments, the VHH domain comprises heavy chain framework regions (e.g., heavy chain framework regions comprised in amino acid sequences encoded by human germline antibody genes) derived from human immunoglobulins, the heavy chain framework regions optionally comprise one or more (e.g., 1, 2, 3, 4, 5, 6, 7, 8, 9 or 10) back mutations from human residues to camelid residues.

In certain embodiments, the first antigen-binding domain comprises: a VHH sequence as shown in SEQ ID NO: 1 or a variant thereof, where the variant has a sequence identity of at least 80%, at least 85%, at least 90%, at least 91%, at least 92%, at least 93%, at least 94%, at least 95%, at least 96%, at least 97%, at least 98%, at least 99%, or 100%, or has a substitution, deletion or addition of one or several amino acids (e.g., substitution, deletion, or addition of 1, 2, 3, 4, 5, 6, 7, 8, 9 or 10 amino acids) as compared to the sequence from which it is derived; preferably, the substitutions are conservative substitutions.

In certain exemplary embodiments, the first antigen-binding domain comprises a VHH as shown in SEQ ID NO: 1.

In certain embodiments, the second antigen-binding domain comprises the antibody or antigen-binding fragment thereof (e.g., heavy chain variable region and/or light chain variable region thereof) that specifically binds to VEGF.

In certain embodiments, the antibody that specifically binds to VEGF is selected from Bevacizumab or its variants, and Ranibizumab or its variants.

In certain embodiments, the second antigen-binding domain comprises the heavy chain variable region (VH) and light chain variable region (VL) derived from Bevacizumab or its variants, wherein:
(i) the VH comprises CDR-H1 as shown in SEQ ID NO: 7, CDR-H2 as shown in SEQ ID NO: 8 and CDR-H3 as shown in SEQ ID NO: 9; the VL comprises CDR-L1 as shown in SEQ ID NO: 10, CDR-L2 as shown in SEQ ID NO: 11, and CDR-L3 as shown in SEQ ID NO: 12;
(ii) the VH comprises: CDR-H1, CDR-H2, and CDR-H3 as contained in the VH as shown in SEQ ID NO: 5; the VL comprises: CDR-L1, CDR-L2, and CDR-L3 as contained in the VL as shown in SEQ ID NO: 6; preferably, the CDRs are determined by the Kabat, IMGT, or Chothia numbering systems.

In certain embodiments, the VH and/or VL further comprise framework regions derived from mammalian (e.g., human or murine) immunoglobulins. In certain embodiments, the VH and/or VL further comprise framework regions derived from human immunoglobulins.

In certain embodiments, the VH comprises the sequence as shown in SEQ ID NO: 5 or a variant thereof, and/or the VL comprises the sequence as shown in SEQ ID NO: 6 or a variant thereof; wherein the variant has a sequence identity of at least 80%, at least 85%, at least 90%, at least 91%, at least 92%, at least 93%, at least 94%, at least 95%, at least 96%, at least 97%, at least 98%, at least 99%, or 100%, or has a substitution, deletion or addition of one or several amino acids (e.g., substitution, deletion, or addition of 1, 2, 3, 4 or 5 amino acids) as compared to the sequence from which it is derived; preferably, the substitutions are conservative substitutions.

In certain exemplary embodiments, the second antigen-binding domain comprises a VH as shown in SEQ ID NO: 5 and a VL as shown in SEQ ID NO: 6.

In certain embodiments, the second antigen-binding domain comprises a heavy chain variable region (VH) and a light chain variable region (VL) derived from Ranibizumab or a variant thereof, wherein:
(i) the VH comprises CDR-H1 as shown in SEQ ID NO: 15, CDR-H2 as shown in SEQ ID NO: 16, and CDR-H3 as shown in SEQ ID NO: 17; the VL comprises CDR-L1 as shown in SEQ ID NO: 18, CDR-L2 as shown in SEQ ID NO: 19, and CDR-L3 as shown in SEQ ID NO: 20; or
(ii) the VH comprises: CDR-H1, CDR-H2, and CDR-H3 as contained in the VH as shown in SEQ ID NO: 13, and the VL comprises: CDR-L1, CDR-L2, and CDR-L3 as contained in the VL as shown in SEQ ID NO: 14; preferably, the CDRs are determined by the Kabat, IMGT, or Chothia numbering systems.

In certain embodiments, the VH and/or VL further comprise framework regions derived from a mammalian (e.g., human or mouse) immunoglobulin.

In certain embodiments, the VH comprises the sequence as shown in SEQ ID NO: 13 or a variant thereof, and/or the VL comprises the sequence as shown in SEQ ID NO: 14 or a variant thereof; the variant has a sequence identity of at least 80%, at least 85%, at least 90%, at least 91%, at least 92%, at least 93%, at least 94%, at least 95%, at least 96%, at least 97%, at least 98%, at least 99%, or 100%, or has a substitution, deletion or addition of one or more amino acids (e.g., substitution, deletions or additions of 1, 2, 3, 4 or 5 amino acids) as compared to the sequence from which it is derived; preferably, the substitutions are conservative substitutions.

In certain exemplary embodiments, the second antigen-binding domain comprises the VH as shown in SEQ ID NO: 13 and the VL as shown in SEQ ID NO: 14.

In certain embodiments, unless otherwise specified, the CDRs contained in the first antigen-binding domain and second antigen-binding domain are determined by the Kabat numbering system.

In certain embodiments, the first antigen-binding domain and the second antigen-binding domain are linked optionally via a linker.

In certain embodiments, the linker is a peptide linker, such as a flexible peptide linker.

In certain embodiments, the linker is a peptide linker comprising one or more glycines and/or one or more serines. In certain embodiments, the linker is a flexible peptide linker comprising (G4S) n, where n is 1, 2, 3, or 4, for example, the linker comprises a sequence as shown in SEQ ID NO: 35.

In certain embodiments, the second antigen-binding domain is a full-length antibody (e.g., IgG antibody), an Fv fragment, an Fab fragment, an F(ab')₂ fragment, or an scFv.

In certain embodiments, the second antigen-binding domain comprises a heavy chain and a light chain, the heavy chain comprises a heavy chain variable region (VH) and a heavy chain constant region (CH), and the light chain comprises a light chain variable region (VL) and a light chain constant region (CL). In certain embodiments, the first antigen-binding domain is linked to the N-terminus and/or C-terminus of the heavy chain and/or to the N-terminus and/or C-terminus of the light chain optionally via a linker.

In certain embodiments, the heavy chain variable region (VH) and light chain variable region (VL) are defined as described in any of the aforementioned embodiments.

In certain embodiments, the heavy chain constant region (CH) is of IgG, for example, IgG1, IgG2, IgG3, or IgG4. In certain exemplary embodiments, the heavy chain constant region (CH) comprises a sequence as shown in SEQ ID NO: 21.

In certain embodiments, the Fc domain comprised in the heavy chain constant region (CH) is a natural Fc region, containing an amino acid sequence identical to the amino acid sequence of Fc regions that found in natural. The natural Fc region may exhibit effector functions, such as binding to Fc receptors; C1q binding and complement-dependent cytotoxicity (CDC); antibody-dependent cellular cytotoxicity (ADCC); antibody-dependent cellular phagocytosis (ADCP); downregulation of cell surface receptors (e.g., B cell receptors); and B cell activation.

In certain embodiments, the Fc domain comprised in the heavy chain constant region (CH) may be a modified Fc region, containing amino acid mutations or chemical modifications as compared to the natural Fc region to alter its effector functions. In certain embodiments, the modified Fc region may include a modification that promotes dimerization.

In certain embodiments, the light chain constant region (CL) is κ or λ light chain constant region. In certain exemplary embodiments, the light chain constant region (CL) comprises a sequence as shown in SEQ ID NO: 22.

In certain embodiments, the second antigen-binding domain comprises at least one heavy chain and at least one light chain, and the first antigen-binding domain is linked to the N-terminus or C-terminus of the heavy chain or to the N-terminus of the light chain optionally via a linker.

In certain embodiments, the second antigen-binding domain comprises two identical heavy chains and two identical light chains, and the first antigen-binding domain is linked to the N-terminus or C-terminus of the two heavy chains, or to the N-terminus of the two light chains optionally via a linker.

In certain embodiments, the multispecific antibody comprises a first peptide chain and a second peptide chain, wherein:
(1) the first peptide chain comprises the following structure: [VHH]-[L]-[VH]-[CH], and the second peptide chain comprises the following structure: [VL]-[CL];
(2) the first peptide chain comprises the following structure: [VH]-[CH], and the second peptide chain comprises the following structure: [VHH]-[L]-[VL]-[CL]; or
(3) the first peptide chain comprises the following structure: [VH]-[CH]-[L]-[VHH], and the second peptide chain comprises the following structure: [VL]-[CL];
wherein, [L] is a peptide linker.

In certain exemplary embodiments, the multispecific antibody comprises:
(1) a first peptide chain comprising a sequence as shown in SEQ ID NO: 23, and a second peptide chain comprising a sequence as shown in SEQ ID NO: 24;
(2) a first peptide chain comprising a sequence as shown in SEQ ID NO: 25, and a second peptide chain comprising a sequence as shown in SEQ ID NO: 26;
(3) a first peptide chain comprising a sequence as shown in SEQ ID NO: 27, and a second peptide chain comprising a sequence as shown in SEQ ID NO: 28;
(4) a first peptide chain comprising a sequence as shown in SEQ ID NO: 29, and a second peptide chain comprising a sequence as shown in SEQ ID NO: 30;
(5) a first peptide chain comprising a sequence as shown in SEQ ID NO: 31, and a second peptide chain comprising a sequence as shown in SEQ ID NO: 32; or
(6) a first peptide chain comprising a sequence as shown in SEQ ID NO: 33, and a second peptide chain comprising a sequence as shown in SEQ ID NO: 34.

In certain embodiments, the multispecific antibody is a bispecific antibody.

In certain embodiments, the multispecific antibody further includes at least one additional antigen-binding domain targeting an antigen different from those bounded by the first and second antigen-binding domains, enabling binding to at least three or more distinct binding sites and/or target molecules. In certain embodiments, the multispecific antibody is a trispecific or tetraspecific antibody.

### Preparation of Antibody

The multispecific antibody of the present invention can be prepared by various methods known in the art, such as genetic engineering and recombinant technology. For example, the multispecific antibody of the present invention can be produced by co-expressing multiple polynucleotides encoding the various polypeptide chains of the multispecific antibody. The polypeptide chains produced by co-expression can be combined, for example, via disulfide bonds or other means to form a functional multispecific antibody.

In another aspect, the present invention provides an isolated nucleic acid molecule, which comprise a nucleotide sequence encoding the multispecific antibody of the present invention or at least one polypeptide chain thereof.

In some embodiments, the isolated nucleic acid molecule comprises nucleotide sequences each encoding a polypeptide chain of the multispecific antibody of the present invention, and the nucleotide sequences are present on the same isolated nucleic acid molecule or different isolated nucleic acid molecules.

In another aspect, the present invention provides a vector (e.g., cloning vector or expression vector) comprising the above-described isolated nucleic acid molecule.

In some embodiments, the vector comprises nucleotide sequences each encoding a polypeptide chain of the multispecific antibody of the present invention, and the nucleotide sequences are present on the same vector or different vectors. For example, the vectors of the present invention may comprise a first vector comprising the nucleotide sequence encoding the first polypeptide chain, and a second vector comprising the nucleotide sequence encoding the second polypeptide chain.

**In** another aspect, the present invention provides a host cell comprising the nucleic acid molecule or vector as described above. Such host cells include, but are not limited to, prokaryotic cells such as bacterial cells (e.g., *E. coli* cells), and eukaryotic cells such as fungal cells (e.g., yeast cells), insect cells, plant cells, and animal cells (e.g., mammalian cells, such as mouse cells, human cells, etc.).

**In** another aspect, the present invention provides methods for preparing the multispecific antibody of the present invention, comprising culturing the above-described host cells under conditions that allow for protein expression, and recovering the multispecific antibody from the culture of the cultured host cells.

### Pharmaceutical Composition

**In** another aspect, the present invention provides a pharmaceutical composition comprising the multispecific antibody of the present invention, the isolated nucleic acid molecule of the present invention, the vector of the present invention or the host cell of the present invention, and a pharmaceutically acceptable carrier and/or excipient.

**In** some embodiments, the pharmaceutical composition comprises the multispecific antibody of the present invention.

**In** some embodiments, the pharmaceutical composition may further comprise an additional pharmaceutically active agent.

**In** some embodiments, the additional pharmaceutically active agent is an anti-tumor agent.

**In** some embodiments, in the pharmaceutical composition, the multispecific antibody, isolated nucleic acid molecule, vector or host cell of the present invention, and the additional pharmaceutically active agent may be provided as separate or mixed components. Thus, the multispecific antibody, isolated nucleic acid molecule, vector or host cell of the present invention, and the additional pharmaceutically active agent may be administered simultaneously, separately or sequentially.

The multispecific antibody, isolated nucleic acid molecule, vector or host cell of the present invention, or the pharmaceutical composition of the present invention, can be formulated into any dosage form known in the medical field, such as tablets, pills, suspensions, emulsions, solutions, gels, capsules, powders, granules, elixirs, lozenges, suppositories, injections (including injectable solutions, sterile powders for injection, and injectable concentrated solutions), inhalers, sprays and the like. The preferred dosage form depends on the intended route of administration and therapeutic use.

A preferred dosage form is an injectable formulation. Such injectable formulations can be sterile injectable solutions. For example, sterile injectable solutions may be prepared by incorporating the required dose of the multispecific antibody of the present invention into an appropriate solvent, and optionally incorporating other desired components (including, but not limited to, pH adjusters, surfactants, adjuvants, ionic strength enhancers, isotonic agents, preservatives, diluents or any combination thereof), followed by sterile filtration. Furthermore, sterile injectable solutions can be prepared as sterile lyophilized powders (e.g., by vacuum drying or freeze-drying) for storage and use. Such sterile lyophilized powders may be reconstituted before use in a suitable carrier, such as water for injection (WFI), bacteriostatic water for injection (BWFI), saline solution (e.g., 0.9% (w/v) NaCl), glucose solution (e.g., 5% glucose), surfactant-containing solution (e.g., 0.01% polysorbate 20), pH buffered solution (e.g., phosphate-buffered solution), Ringer's solution or any combination thereof.

In some exemplary embodiments, the pharmaceutical composition of the present invention comprises a sterile injectable liquid (such as an aqueous or non-aqueous suspension or solution). In some exemplary embodiments, the sterile injectable liquid is selected from water for injection (WFI), bacteriostatic water for injection (BWFI), saline solution (e.g., 0.9% (w/v) NaCl), glucose solution (e.g., 5% glucose), surfactant-containing solution (e.g., 0.01% polysorbate 20), pH buffered solution (e.g., phosphate-buffered solution), Ringer's solution or any combination thereof.

The multispecific antibody, isolated nucleic acid molecule, vector or host cell of the present invention, or the pharmaceutical composition of the present invention, may be administered by any suitable method known in the art, including, but not limited to, oral, buccal, sublingual, ocular, topical, parenteral, rectal, intrathecal, intracytoplasmic, inguinal, intravesical, topical (e.g., powders, ointments, or drops), or nasal routes. However, for many therapeutic uses, the preferred route of administration is parenteral (e.g., intravenous injection or bolus, subcutaneous injection, intraperitoneal injection, intramuscular injection). Persons skilled in the art will understand that the route and/or method of administration will vary depending on the intended purpose. In some embodiments, the multispecific antibody, isolated nucleic acid molecule, vector or host cell of the present invention, or the pharmaceutical composition of the present invention, are administered by intravenous injection or bolus.

The pharmaceutical composition of the present invention may include a "therapeutically effective amount" or a "prophylactically effective amount" of the multispecific antibody, isolated nucleic acid molecule, vector or host cell of the present invention. A "prophylactically effective amount" refers to an amount sufficient to prevent, inhibit, or delay the onset of a disease. A "therapeutically effective amount" refers to an amount sufficient to cure or at least partially inhibit the disease in a patient already suffering from the disease, along with its complications. The therapeutically effective amount may vary depending on factors such as the severity of the disease being treated, the general state of the patient's immune system, the patient's general condition, such as age, weight, and gender, the method of administration and other concomitant treatments.

### Therapeutic Use

**In** one aspect, the present invention provides a method for preventing and/or treating a tumor, comprising administering the multispecific antibody, isolated nucleic acid molecule, vector, host cell or pharmaceutical composition of the present invention to a subject in need. The invention also provides the use of the multispecific antibody, the isolated nucleic acid molecule, the vector, the host cell or the pharmaceutical composition of the present invention for preventing and/or treating a tumor, or for manufacturing of a medicament for preventing and/or treating a tumor.

**In** certain embodiments, the tumor is selected from solid tumors.

**In** certain embodiments, the tumor is selected from lung cancer (e.g., non-small cell lung cancer or small cell lung cancer), liver cancer (e.g., hepatocellular carcinoma), kidney cancer (e.g., renal cell carcinoma), stomach cancer, ovarian cancer, cervical cancer, endometrial cancer, breast cancer (e.g., triple-negative breast cancer), colorectal cancer and brain tumors (e.g., glioblastoma).

**In** certain embodiments, an agent provided by the present invention is administered in combination with a second therapeutic agent (e.g., an antitumor agent) or treatment (e.g., surgery, chemotherapy, radiotherapy, targeted therapy, immunotherapy, hormone therapy, gene therapy or palliative care), and selected from the multispecific antibody, isolated nucleic acid molecule, vector, host cell or pharmaceutical composition of the present invention. The second therapeutic agent or treatment may be administered before, simultaneously or after the administration of the aforementioned agent of the invention.

### Definition of Terms

In this invention, unless otherwise specified, scientific and technical terms used herein have the meanings commonly understood by those skilled in the arts. Furthermore, virology, biochemistry and immunology laboratory procedures described herein refer to conventional methods widely used in the respective arts. To further clarify the present invention, definition and explanation of relevant terms are provided below.

As used herein, the term "antibody" refers to molecules that specifically bind to an epitope and may encompass various antibody structures, provided they exhibit the desired antigen-binding activity. Typically, an antibody can be an immunoglobulin molecule composed of two pairs of polypeptide chains (each pair having one light chain (LC) and one heavy chain (HC)). Antibody light chains may be classified as κ (kappa) or λ (lambda) light chains. Heavy chains may be classified as µ, δ, γ, α, or ε, which define the isotypes of the antibody as IgM, IgD, IgG, IgA, or IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of the classical complement system (C1q). The VH and VL regions can further be subdivided into hypervariable regions known as complementarity-determining regions (CDRs), interspersed with more conserved framework regions (FRs). Each VH and VL consists of three CDRs and four FRs, arranged in the following order from the N-terminus to the C-terminus: FR1, CDR1, FR2, CDR2, FR3, CDR3 and FR4. The variable regions (VH and VL) of each heavy chain/light chain pair form the antigen-binding site.

As used herein, the term "complementarity-determining region" or "CDR" refers to the amino acid residues in the variable region of an antibody responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed., Public Health Service, National Institutes of Health, Bethesda, Md., 1991), the Chothia numbering system (Chothia & Lesk, J. Mol. Biol. 196:901-917, 1987; Chothia et al., Nature 342:878-883, 1989), or the IMGT numbering system (Lefranc et al., Dev. Comp. Immunol. 27:55-77, 2003). For a given antibody, one skilled in the art will readily identify the CDRs defined by each numbering system. Moreover, the correspondence between different numbering systems is well known to those skilled in the art (Lefranc et al., Dev. Comp. Immunol. 27:55-77, 2003).

In the present invention, the CDRs contained in the antibody or antigen-binding fragments thereof can be determined according to various numbering systems known in the art. In certain embodiments, the CDRs contained in the antibody or antigen-binding fragment thereof of the present invention are preferably determined by the IMGT, Kabat or Chothia numbering systems. In certain embodiments, unless otherwise specified, the CDRs contained in the antibody or antigen-binding fragment thereof of the present invention are preferably determined by the Kabat numbering system.

As used herein, the term "framework region" or "FR" refers to those amino acid residues in the variable region of an antibody other than the CDR residues defined above.

The term "antibody" is not limited by any specific method of antibody production. For example, it includes recombinant antibodies, monoclonal antibodies and polyclonal antibodies. Antibodies may be an antibody of different isotypes, such as IgG (e.g., IgG1, IgG2, IgG3, or IgG4 subtype), IgA1, IgA2, IgD, IgE or IgM antibody.

As used herein, the term "multispecific antibody" refers to an antibody capable of specific binding to at least two (e.g., two, three or four) different antigens (or epitopes). Multispecific antibodies comprise multiple antigen-binding domains that specific bind to different antigens (or epitopes), enabling them to interact with at least two distinct binding sites and/or target molecules. Each antigen-binding domain of a multispecific antibody may be independently selected from full-length antibodies (e.g., IgG antibodies) or antigen-binding fragments thereof (e.g., Fv fragments, Fab fragments, (Fab')₂ fragments or scFv). In some cases, the antigen-binding domains are connected by a peptide linker. In certain embodiments, the multispecific antibody of the present invention may be a bispecific antibody. In certain embodiments, the multispecific antibody may be a trispecific or tetraspecific antibody.

As used herein, the term "single-domain antibody (sdAb)" has the meaning commonly understood by those skilled in the art, which refers to an antibody fragment composed of a single monomeric variable antibody domain (e.g., a single heavy chain variable region). These antibodies are typically derived from the variable region of heavy chain antibodies (e.g., camelids or sharks). A typical sdAb consists of four framework regions and three CDRs, arranged in an FR1-CDR1-FR2-CDR2-FR3-CDR3-FR4 structure. The sdAb may be truncated at the N- or C-terminus to comprise only part of FR1 and/or FR4, or one or two of those framework regions may be absent, provided the antigen-binding activity and specificity are substantially maintained. A single-domain antibody is also referred to as a nanobody, and the terms can be used interchangeably. The variable region of a single-domain antibody or nanobody is referred to as a VHH domain.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety" See generally, Fundamental Immunology, Ch. 7 (Paul, W., ed., 2nd ed., Raven Press, N.Y., 1989), which is incorporated herein by reference in its entirety for all purposes. Antigen-binding fragments of an antibody can be obtained by recombinant DNA technology or enzymatic or chemical cleavage of the full-length antibody. Non-limiting examples of antigen-binding fragments include Fab, Fab', (Fab')₂, Fv, disulfide-linked Fv, scFv, di-scFv, (scFv)₂ and such polypeptides, which contain at least a portion of an antibody that is sufficient to confer specificity to the polypeptides with antigen-binding capability.

As used herein, the term "Fab" refers to an antibody fragment consisting of a light chain comprising VL and CL and a heavy chain fragment comprising VH and CH1; the term "(Fab')₂" refers to an antibody fragment comprising two Fab fragments connected by a disulfide bridge at the hinge region; the term "Fab'" refers to a fragment obtained by reducing the disulfide bond linking the two heavy chain fragments in the F(ab')² fragment, which consists of a complete light chain and an Fd fragment of heavy chain (consisting of VH and CH1 domains).

As used herein, the term "Fv" refers to an antibody fragment consisting of the variable regions of the light chain (VL) and heavy chain (VH) of a single arm of an antibody. Fv fragments are generally considered to be the smallest antibody fragments capable of forming a complete antigen binding site.

As used herein, the term "scFv" refers to a single polypeptide chain comprising the VL and VH regions, wherein the VL and VH regions are connected by a linker. Such scFv molecules may have a general structure: NH₂-VL-linker-VH-COOH or NH₂-VH-linker-VL-COOH. Suitable linkers from prior art typically consist of repeated GGGGS amino acid sequences or variants thereof. For example, a linker having the amino acid sequence (GGGGS)₄ may be used, but variants thereof may also be used. In some cases, a disulfide bond may be present between the VH and VL of the scFv. In some cases, scFvs may form a di-scFv, which refers to antibodies formed by connecting two or more single scFvs in series. In some cases, scFvs may form (scFv)₂, which refers to antibodies formed by linking two or more individual scFvs in parallel.

As used herein, the term "variant" in the context of polypeptides (including a polypeptide) refers to a polypeptide or peptide comprising an amino acid sequence that has been altered by the introduction of substitution, deletion, or addition of amino acid residues. In some cases, the term "variant" also refers to a polypeptide or peptide that has been modified (i.e., by covalently attaching any type of molecule to the polypeptide or peptide). For example, but not limited to, a polypeptide can be modified, for example, by glycosylation, acetylation, PEGylation, phosphorylation, amidation, derivatization by known protecting /blocking groups, proteolytic cleavage, attachment to cell ligands, or other proteins. Derivatized polypeptides or peptides can be produced by chemical modification using techniques known to those skilled in the art, including, but not limited to, specific chemical cleavage, acetylation, formylation, and metabolic synthesis of tunicamycin, etc. In addition, the variant has similar, identical or improved functions to the polypeptide or peptide from which it is derived.

As used herein, the term "specific binding" refers to a non-random interaction between two molecules, such as the interaction between an antibody and its target antigen. The strength or affinity of a specific binding interaction can be expressed by the equilibrium dissociation constant (KD) of the interaction. The smaller the equilibrium dissociation constant, the tighter the antibody-antigen binding and the higher the affinity between the antibody and the antigen. The specific binding properties between two molecules can be measured using methods well known in the art, such as surface plasmon resonance (SPR) using Biacore or bio-layer interferometry (BLI) or Kinexa to measure dissociation constants.

As used herein, the term "vector" refers to a nucleic acid delivery tool into which a polynucleotide can be inserted. When a vector can express the protein encoded by the inserted polynucleotide, the vector is called an expression vector. Vectors can be introduced into host cells by transformation, transduction or transfection, allowing the genetic material elements carried by the vector to be expressed in the host cell. Vectors are well known to those skilled in the art, and include but are not limited to: plasmids, phagemids, cosmid vectors, artificial chromosomes (e.g., yeast artificial chromosomes (YACs), bacterial artificial chromosomes (BACs), or P1-derived artificial chromosomes (PACs)), bacteriophages such as λ phage or M13 phage, and animal viruses. Animal viruses that may be used as vectors include but are not limited to, retroviruses (including lentiviruses), adenoviruses, adeno-associated viruses, herpes viruses (such as herpes simplex virus), poxviruses, baculoviruses, papillomaviruses, and polyomaviruses (such as SV40). A vector can contain a variety of elements that control expression, including but not limited to promoter sequences, transcription initiation sequences, enhancer sequences, selectable elements, and reporter genes. Additionally, vectors may contain replication origins.

As used herein, the term "host cell" refers to cells that can be used to introduce a vector, including but not limited to, prokaryotic cells such as *Escherichia coli* or *Bacillus subtilis,* eukaryotic cells such as fungal cells (e.g., yeast or *Aspergillus*)*,* insect cells such as S2 cells or Sf9 cells, or animal cells such as fibroblasts, CHO cells, COS cells, NSO cells, HeLa cells, BHK cells, HEK 293 cells, or human cells, and immune cells (e.g., T lymphocytes, NK cells, monocytes, macrophages, or dendritic cells). Host cells may include individual cells or cell populations.

As used herein, the term "identity" refers to the degree of sequence matching between two polypeptides or two nucleic acids. When a position in the compared sequence is occupied by the same base or amino acid monomer subunit (for example, a position in each of two DNA molecules is occupied by adenine, or a position in each of two peptides is occupied by lysine), then each molecule is the same at that position. The "percentage identity" between two sequences is the ratio of matching positions to the total number of positions compared, expressed as a percentage. For example, if six out of ten positions match, the sequences have 60% identity. DNA sequences CTGACT and CAGGTT share 50% identity (three matching positions out of six). Typically, sequences are compared by alignment to maximize identity. Such alignments can be achieved using methods such as Needleman-Wunsch algorithms implemented in computer programs like ALIGN (DNAstar, Inc.) (J Mol Biol. 1970 Mar;48(3):443-53.). The percentage identity between amino acid sequences can also be calculated using algorithms such as those of E. Meyers and W. Miller, available in the ALIGN program (version 2.0), using a PAM120 weight residue table, gap length penalties of 12, and gap penalties of 4 (Comput. Appl Biosci., 4:11-17 (1988)). In addition, the Needleman and Wunsch (J Mol Biol. 48:44-543 (1970)) algorithms integrated into the GAP program of the GCG software package (available on www.gcg. com) can be used to determine the percentage identity between two amino acid sequences using Blossum 62 matrix or PAM250 matrix, as well as gap weights of 16, 14, 12, 10, 8, 6, or 4 and length weights of 1, 2, 3, 4, 5, or 6.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (Brummell et al., Biochem. 32:1180-1187, 1993; Kobayashi et al., Protein Eng. 12(10):879-884, 1999; and Burks et al., Proc. Natl Acad. Sci. USA 94:412-417, 1997).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to carriers and/or excipients that are pharmacologically and/or physiologically compatible with a subject and an active ingredient (see, for example, Remington's Pharmaceutical Sciences. Edited by Gennaro AR, 19th ed. Pennsylvania: Mack Publishing Company, 1995), including, but not limited to: pH regulators, surfactants, adjuvants, ionic strength enhancers, diluents, agents for maintaining osmotic pressure, delayed absorption agents and preservatives. For example, pH regulators includes, but is not limited to, phosphate buffer. The surfactant includes, but is not limited to, cationic, anionic or nonionic surfactant, such as Tween-80. The ionic strength enhancer includes, but is not limited to, sodium chloride. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), glucose solution (e.g., 5% glucose), a solution containing a surfactant (e.g., 0.01% polysorbate 20), a pH buffer solution (e.g., a phosphate buffer solution), Ringer's solution, and any combination thereof

As used herein, the term "prevention" refers to a method implemented to prevent or delay the occurrence of a disease, disorder, or symptom in a subject. As used herein, the term "treatment" refers to a method implemented to obtain a beneficial or desired clinical result. For the purposes of the present invention, beneficial or desired clinical results include (but are not limited to) alleviation of symptoms, reduction of the extent of the disease, stabilization (i.e., no longer worsening) of the state of the disease, delay or slowing the progression of the disease, improvement or alleviation of the state of the disease, and relief of symptoms (whether partial or complete), whether detectable or undetectable. In addition, "treatment" can also refer to prolonging survival compared to the expected survival (if not receiving treatment).

As used herein, the term "subject" refers to a mammal, such as a primate, including a human. In certain embodiments, the subject (e.g., a human) has a tumor.

As used herein, the terms "cancer" and "tumor" are used interchangeably and refer to a broad class of diseases characterized by uncontrolled growth of abnormal cells in the body. Unregulated cell division can lead to the formation of malignant tumors or invasive cells in surrounding tissues and may metastasize to distant parts of the body via the lymphatic system or bloodstream. Cancer includes both benign and malignant cancers, as well as dormant tumors or micrometastases. Cancer includes solid tumors as well as hematologic malignancies.

### Beneficial effects of the invention

The present invention provides a multispecific antibody targeting PD-1 and VEGF, which is capable of specifically blocking PD-1 and VEGF signaling pathways, thereby producing a synergistic anti-tumor effect, while reducing the incidence of adverse reactions and making clinical medication more convenient. The multispecific antibody of the present invention has important clinical value.

The following will describe the embodiments of the present invention in detail in conjunction with the accompanying drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows the results of PD1-blocking activity in a cell-based experiment.
Fig. 2 shows the results of VEGF-blocking activity in a cell-based experiment.
Fig. 3 shows the changes in tumor volume in an in vivo anti-tumor experiment.
Fig. 4 shows the changes in mouse body weight in an in vivo anti-tumor experiment.

### Sequence information

The information of the sequences involved in the present invention is provided in the table below.Table 1. Sequence information

| SEQ ID NO: | Description | Sequence |
|---|---|---|
| 1 | Anti-PD-1 sdAb VHH | QVQLVESGGGLVQPGGSLRLSCATSGSRRSIYAMGWFRQAPGKGL EFVAGIGWAYATTYYADSVKGRFTISRDNTKNTLYLQMNSLRAEDT AVYYCAADLDHSGFDYWGQGTLVTVSS |
| 2 | Anti-PD-1 sdAb CDR1 | IYAMG |
| 3 | Anti-PD-1 sdAb CDR2 | GIGWAYATTYYADSVKG |
| 4 | Anti-PD-1 sdAb CDR3 | DLDHSGFDY |
| 5 | Bevacizumab (mutant) VH | EVQLVQSGGGVVQPGGSLRLSCAASGYTFTNYGMNWVRQAPGK GLEWVGWINTYTGEPTYAADFKRRFTFSLDTSKSTAYLQMNSLRA EDTAVYYCAKYPHYYGSSHWYFDVWGQGTLVTVSS |
| 6 | Bevacizumab (mutant) VL | |
| 7 | Bevacizumab (mutant) CDR-H1 | NYGMN |
| 8 | Bevacizumab (mutant) CDR-H2 | WINTYTGEPTYAADFKR |
| 9 | Bevacizumab (mutant) CDR-H3 | YPHYYGSSHWYFDV |
| 10 | Bevacizumab (mutant) CDR-L1 | SASQDISNYLN |
| 11 | Bevacizumab (mutant) CDR-L2 | FTSSLHS |
| 12 | Bevacizumab (mutant) CDR-L3 | QQYSTVPWT |
| 13 | Ranibizumab VH | |
| 14 | Ranibizumab VL | |
| 15 | Ranibizumab CDR-H1 | HYGMN |
| 16 | Ranibizumab CDR-H2 | WINTYTGEPTYAADFKR |
| 17 | Ranibizumab CDR-H3 | YPYYYGTSHWYFDV |
| 18 | Ranibizumab CDR-L1 | SASQDISNYLN |
| 19 | Ranibizumab CDR-L2 | FTSSLHS |
| 20 | Ranibizumab CDR-L3 | QQYSTVPWT |
| 21 | Heavy chain constant region | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTK VDKKVEPKSCDKTHTCPPCPAPEAAGGPSVFLFPPKPKDTLMISRTP EVTCVVVDVSHEDPEVKFNWYVDGVEVHNAKTKPREEQYNSTY RVVSVLTVLHQDWLNGKEYKCKVSNKALPAPIEKTISKAKGQPRE PQVYTLPPSREEMTKNQVSLTCLVKGFYPSDIAVEWESNGQPENNY KTTPPVLDSDGSFFLYSKLTVDKSRWQQGNVFSCSVMHEALHNHY TQKSLSLSPGK |
| 22 | Light chain constant region | |
| | | |
| 23 | 162-17-HC | QVQLVESGGGLVQPGGSLRLSCATSGSRRSIYAMGWFRQAPGKGL EFVAGIGWAYATTYYADSVKGRFTISRDNTKNTLYLQMNSLRAEDT AVYYCAADLDHSGFDYWGQGTLVTVSSGGGGSGGGGSGGGGSEV QLVQSGGGVVQPGGSLRLSCAASGYTFTNYGMNWVRQAPGKGLE WVGWINTYTGEPTYAADFKRRFTFSLDTSKSTAYLQMNSLRAEDT AVYYCAKYPHYYGSSHWYFDVWGQGTLVTVSSASTKGPSVFPLA PSSKSTSGGTAALGCLVKDYFPEPVTVSWNSGALTSGVHTFPAVLQ SSGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLTCLVKGFYPSDIAVEWESNGQPENNYKTTPPVLDSDG SFFLYSKLTVDKSRWQQGNVFSCSVMHEALHNHYTQKSLSLSPGK |
| 24 | 162-17-LC | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 25 | 162-18-HC | EVQLVQSGGGVVQPGGSLRLSCAASGYTFTNYGMNWVRQAPGK GLEWVGWINTYTGEPTYAADFKRRFTFSLDTSKSTAYLQMNSLRA EDTAVYYCAKYPHYYGSSHWYFDVWGQGTLVTVSSASTKGPSVF PLAPSSKSTSGGTAALGCLVKDYFPEPVTVSWNSGALTSGVHTFPA VLQSSGLYSLSSVVTVPSSSLGTQTYICNVNHKPSNTKVDKKVEPK SCDKTHTCPPCPAPEAAGGPSVFLFPPKPKDTLMISRTPEVTCVVVD VSHEDPEVKFNWYVDGVEVHNAKTKPREEQYNSTYRVVSVLTVL HQDWLNGKEYKCKVSNKALPAPIEKTISKAKGQPREPQVYTLPPS REEMTKNQVSLTCLVKGFYPSDIAVEWESNGQPENNYKTTPPVLDS DGSFFLYSKLTVDKSRWQQGNVFSCSVMHEALHNHYTQKSLSLSP GK |
| 26 | 162-18-LC | QVQLVESGGGLVQPGGSLRLSCATSGSRRSIYAMGWFRQAPGKGL EFVAGIGWAYATTYYADSVKGRFTISRDNTKNTLYLQMNSLRAEDT AVYYCAADLDHSGFDYWGQGTLVTVSSGGGGSGGGGSGGGGSDI QMTQSPSSLSASVGDRVTITCSASQDISNYLNWYQQKPGKAPKVLI YFTSSLHSGVPSRFSGSGSGTDFTLTISSLQPEDFATYYCQQYSTVP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC |
| 27 | 162-19-HC | EVQLVQSGGGVVQPGGSLRLSCAASGYTFTNYGMNWVRQAPGK GLEWVGWINTYTGEPTYAADFKRRFTFSLDTSKSTAYLQMNSLRA EDTAVYYCAKYPHYYGSSHWYFDVWGQGTLVTVSSASTKGPSVF PLAPSSKSTSGGTAALGCLVKDYFPEPVTVSWNSGALTSGVHTFPA VLQSSGLYSLSSVVTVPSSSLGTQTYICNVNHKPSNTKVDKKVEPK SCDKTHTCPPCPAPEAAGGPSVFLFPPKPKDTLMISRTPEVTCVVVD VSHEDPEVKFNWYVDGVEVHNAKTKPREEQYNSTYRVVSVLTVL HQDWLNGKEYKCKVSNKALPAPIEKTISKAKGQPREPQVYTLPPS REEMTKNQVSLTCLVKGFYPSDIAVEWESNGQPENNYKTTPPVLDS DGSFFLYSKLTVDKSRWQQGNVFSCSVMHEALHNHYTQKSLSLSP GKGGGGSGGGGSGGGGSQVQLVESGGGLVQPGGSLRLSCATSGSR RSIYAMGWFRQAPGKGLEFVAGIGWAYATTYYADSVKGRFTISRDN TKNTLYLQMNSLRAEDTAVYYCAADLDHSGFDYWGQGTLVTVSS |
| 28 | 162-19-LC | DIQMTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 29 | 162-20-HC | QVQLVESGGGLVQPGGSLRLSCATSGSRRSIYAMGWFRQAPGKGL EFVAGIGWAYATTYYADSVKGRFTISRDNTKNTLYLQMNSLRAEDT |
| | | AVYYCAADLDHSGFDYWGQGTLVTVSSGGGGSGGGGSGGGGSEV QLVESGGGLVQPGGSLRLSCAASGYDFTHYGMNWVRQAPGKGLE WVGWINTYTGEPTYAADFKRRFTFSLDTSKSTAYLQMNSLRAEDT AVYYCAKYPYYYGTSHWYFDVWGQGTLVTVSSASTKGPSVFPLA PSSKSTSGGTAALGCLVKDYFPEPVTVSWNSGALTSGVHTFPAVLQ SSGLYSLSSVVTVPSSSLGTQTYICNVNHKPSNTKVDKKVEPKSCD KTHTCPPCPAPEAAGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSREE MTKNQVSLTCLVKGFYPSDIAVEWESNGQPENNYKTTPPVLDSDG SFFLYSKLTVDKSRWQQGNVFSCSVMHEALHNHYTQKSLSLSPGK |
| 30 | 162-20-LC | DIQLTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 31 | 162-21-HC | EVQLVESGGGLVQPGGSLRLSCAASGYDFTHYGMNWVRQAPGKG LEWVGWINTYTGEPTYAADFKRRFTFSLDTSKSTAYLQMNSLRAE DTAVYYCAKYPYYYGTSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPEAAGGPSVFLFPPKPKDTLMISRTPEVTCVVVD VSHEDPEVKFNWYVDGVEVHNAKTKPREEQYNSTYRVVSVLTVL HQDWLNGKEYKCKVSNKALPAPIEKTISKAKGQPREPQVYTLPPS REEMTKNQVSLTCLVKGFYPSDIAVEWESNGQPENNYKTTPPVLDS DGSFFLYSKLTVDKSRWQQGNVFSCSVMHEALHNHYTQKSLSLSP GK |
| 32 | 162-21-LC | QVQLVESGGGLVQPGGSLRLSCATSGSRRSIYAMGWFRQAPGKGL EFVAGIGWAYATTYYADSVKGRFTISRDNTKNTLYLQMNSLRAEDT AVYYCAADLDHSGFDYWGQGTLVTVSSGGGGSGGGGSGGGGSDI QLTQSPSSLSASVGDRVTITCSASQDISNYLNWYQQKPGKAPKVLI YFTSSLHSGVPSRFSGSGSGTDFTLTISSLQPEDFATYYCQQYSTVP WTFGQGTKVEIKRTVAAPSVFIFPPSDEQLKSGTASVVCLLNNFYPR EAKVQWKVDNALQSGNSQESVTEQDSKDSTYSLSSTLTLSKADYE KHKVYACEVTHQGLSSPVTKSFNRGEC |
| 33 | 162-22-HC | EVQLVESGGGLVQPGGSLRLSCAASGYDFTHYGMNWVRQAPGKG LEWVGWINTYTGEPTYAADFKRRFTFSLDTSKSTAYLQMNSLRAE DTAVYYCAKYPYYYGTSHWYFDVWGQGTLVTVSSASTKGPSVFP LAPSSKSTSGGTAALGCLVKDYFPEPVTVSWNSGALTSGVHTFPAV LQSSGLYSLSSVVTVPSSSLGTQTYICNVNHKPSNTKVDKKVEPKS CDKTHTCPPCPAPEAAGGPSVFLFPPKPKDTLMISRTPEVTCVVVD VSHEDPEVKFNWYVDGVEVHNAKTKPREEQYNSTYRVVSVLTVL HQDWLNGKEYKCKVSNKALPAPIEKTISKAKGQPREPQVYTLPPS REEMTKNQVSLTCLVKGFYPSDIAVEWESNGQPENNYKTTPPVLDS DGSFFLYSKLTVDKSRWQQGNVFSCSVMHEALHNHYTQKSLSLSP GKGGGGSGGGGSGGGGSQVQLVESGGGLVQPGGSLRLSCATSGSR RSIYAMGWFRQAPGKGLEFVAGIGWAYATTYYADSVKGRFTISRDN TKNTLYLQMNSLRAEDTAVYYCAADLDHSGFDYWGQGTLVTVSS |
| 34 | 162-22-LC | DIQLTQSPSSLSASVGDRVTITCSASQDISNYLNWYQQKPGKAPKV LIYFTSSLHSGVPSRFSGSGSGTDFTLTISSLQPEDFATYYCQQYSTV PWTFGQGTKVEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 35 | Linker | GGGGSGGGGSGGGGS |

### EXAMPLES

The present invention is now described in the following non-limiting examples.

Those skilled in the art will appreciate that the examples describe the present invention by way of example and are not intended to limit the scope sought to be protected by the present application. The experimental methods in the examples, unless otherwise specified, were conventional methods. If the specific conditions were not specified in the examples, the conventional conditions or the conditions recommended by the manufacturer should be followed. If the manufacturer of the reagents or instruments used was not specified, they were all conventional products that could be obtained commercially. The present invention is now described with reference to the following examples that are intended to illustrate the present invention (but not to limit the present invention).

### Example 1 Design and expression of bispecific antibodies

### 1. Preparation of anti-PD-1 single-domain antibody

A healthy adult female alpaca (Alpaca) was immunized with recombinant human PD-1 protein. After the immunization, peripheral blood of the alpaca was collected, lymphocytes were isolated, and total RNA was extracted and reversely transcribed to obtain cDNA to establish an anti-PD-1 single-domain antibody phage display library. VHH single-domain antibodies that had strong binding activity to human PD-1 and were capable of blocking the binding of human PD-L1 to human recombinant PD-1 and competing with pembrolizumab for binding to the same or overlapping PD-1 epitopes were screened. The detailed screening process can be found in the international patent application PCT/CN2022/074550. Finally, a VHH single-domain antibody was screened and obtained, whose VHH sequence was set forth in SEQ ID NO: 1, and according to the definition of the Kabat numbering system, its CDR1 to CDR3 were set forth in SEQ ID NOs: 2-4, respectively.

### 2. Design and expression of bispecific antibodies

Bispecific antibodies were constructed based on the above anti-PD-1 VHH antibody antibody and anti-VEGF antibodies. The anti-VEGF antibodies used were Bevacizumab (mutant sequence from U.S. Patent 7575893) and Ranibizumab sequences. The VH and VL of Bevacizumab (mutant) were set forth in SEQ ID NOs: 5-6, respectively, and according to the definition of the Kabat numbering system, its CDR-H1 to CDR-H3 were set forth in SEQ ID NOs: 7-9, respectively, and its CDR-L1 to CDR-L3 were set forth in SEQ ID NOs: 10-12, respectively. The VH and VL of Ranibizumab were set forth in SEQ ID NOs: 13-14, respectively, and according to the definition of the Kabat numbering system, its CDR-H1 to CDR-H3 were set forth in SEQ ID NOs: 15-17, respectively, and its CDR-L1 to CDR-L3 were set forth in SEQ ID NOs: 18-20, respectively.

Six bispecific antibodies were constructed, with their structures were shown in Table 2. For the 162-17 and 162-20, their heavy chains had the structure shown in [VHH]-[L]-[VH]-[CH], and their light chains had the structure shown in [VL]-[CL]. For the 162-18 and 162-21, their heavy chains had the structure shown in [VH]-[CH], and their light chains had the structure shown in [VHH]-[L]-[VL]-[CL]. For the 162-19 and 162-22, their heavy chains had the structure shown in [VH]-[CH]-[L]-[VHH], and their light chains had the structure shown in [VL]-[CL]. The above [L] all represented the linker as set forth in SEQ ID NO: 35.

**Table 2. Structure and sequence of bispecific antibodies**

| Name | Heavy-chain (SEQ ID NO:) | | | | Light chain (SEQ ID NO:) | | | |
|---|---|---|---|---|---|---|---|---|
| | VH | CH | VHH position | Full-length | VL | CL | VHH position | Full-length |
| 162-17 | 5 | 21 | N-terminal | 23 | 6 | 22 | / | 24 |
| 162-18 | | | / | 25 | | | N-terminal | 26 |
| 162-19 | | | C-terminal | 27 | | | / | 28 |
| 162-20 | 13 | | N-terminal | 29 | 14 | | / | 30 |
| 162-21 | | | / | 31 | | | N-terminal | 32 |
| 162-22 | | | C-terminal | 33 | | | / | 34 |

The nucleic acid molecules encoding each polypeptide chain of the abovementioned bispecific antibodies were constructed into expression vectors and transiently expressed via HEK cells. After expression, the antibodies were purified by Protein A. The expression yield and purity after purification were shown in Table 3.

**Table 3. Expression yield and purity of bispecific antibodies**

| Name | Molecular weight | Isoelectric point | Expression (mg/L) | Concentration (mg/mL) | SEC,280nm |
|---|---|---|---|---|---|
| 162-17 | 172Kda | 8.25 | 127.4 | 3.98 | 97.176% |
| 162-18 | 172Kda | 8.25 | 47.1 | 2.25 | 73.392% |
| 162-19 | 172Kda | 8.25 | 60.9 | 1.74 | 78.133% |
| 162-20 | 172Kda | 7.98 | 135.1 | 2.33 | 93.950% |
| 162-21 | 172Kda | 7.98 | 150.9 | 3.39 | 68.142% |
| 162-22 | 172Kda | 7.98 | 89.0 | 1.87 | 95.290% |

### Example 2. Experiment on activity of blocking PD1

The activities of the bispecific antibodies to block PD-1/PD-L1 signaling pathway were measured in this example.

PDL1-CHO cells (GenScript, Cat# RD00703) were seeded at 3.5×10⁴ cells/well in 96-well white plates (Thermo Cat# 136101), in which the cells were inoculated in the middle 6×10 wells of the 96-well plate, and 200ul of culture medium was added to the surrounding wells. After culture overnight at 37°C with 5% CO₂ for 16-20 hours, on the next day, complete culture medium (RPMI 1640 + 10% FBS) was used to prepare PD1-Jurkat cell fluids (GenScript, Cat#RD00702) with 4×10⁵ cells/ml and the antibody to be tested (initial concentration was 300nM, 4-fold dilution), respectively, and the two were added to the 96-well plate from which the culture medium was discarded, and incubated at 37°C, 5% CO2 for 6 hours, and Fire-LumiTM luciferase detection reagent (GenScript, Cat#L00877C) was added to detect chemical signals. Four-parameter curves were drawn using Graphpad.

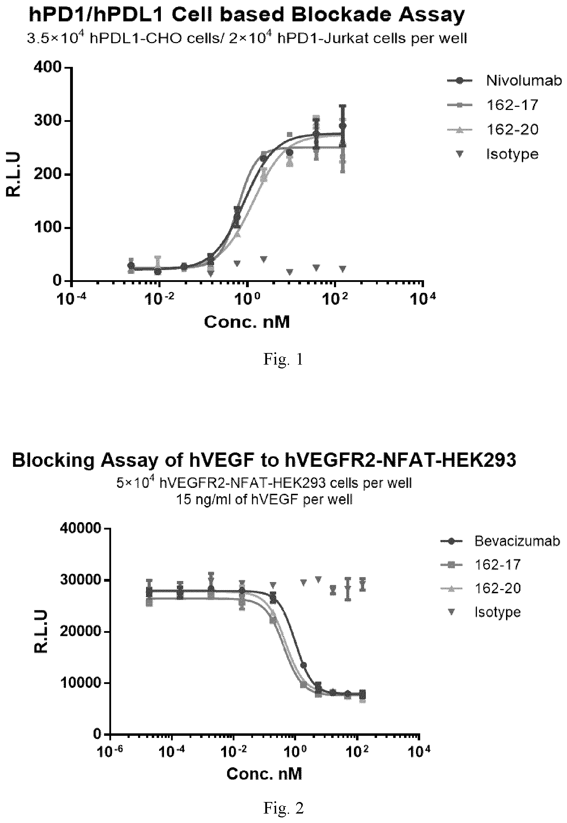

The results were shown in Fig. 1 and Table 4. The bispecific antibodies of the present application had a significant activity to block PD1/PD-L1.

**Table 4. Experiments on activity of blocking PD1**

| | Nivolumab (n=2) | 162-17(n=2) | 162-20 (n=2) |
|---|---|---|---|
| EC50 (nM) | 0.8510 | 0.6073 | 1.356 |
| Top | 276.8 | 250.9 | 274.4 |
| Bottom | 21.68 | 25.03 | 23.62 |

### Example 3. Experiment on activity of blocking VEGF

The activities of the bispecific antibodies to block VEGF signaling pathway were measured in this example.

HEK293 cells overexpressing VEGFR2(GenScript, Cat#RD00704) were seeded into 96-well plates, 5,000 cells per well, and cultured overnight for 16-20 hr. Complete medium (DMEM+10% FBS) was used to prepare 2×VEGF165 (Sino Biological, Cat#11066-HNAH) and the antibody to be tested. The recommended concentration of 2×VEGF165 was 30ng/ml; and the concentration of 2× antibody to be tested was 20ug/ml, and subjected to 3-fold dilution to provide 5 points, and 10-fold dilution to provide 5 points. After preparing the above solutions, the medium was discarded from VEGFR2-HEK293, and then 50ul of VEGF165 and 50ul of the antibody to be tested were immediately added and mixed; incubated at 37°C, 5% CO₂, and 100ul of complete medium was added to the cell-free wells to take the background absorption value. After 6 hours, Fire-Lumi^{™} reagent at 100ul/well was added, and incubated for 5-60 min, and chemical signals were detected.

The results were shown in Fig. 2 and Table 5. The bispecific antibodies of the present application had a significant activity to block VEGF signaling pathway.

**Table 5. Experiments on activity of blocking VEGF**

| | Bevacizumab(n=2) | 162-17(n=2) | 162-20(n=2) |
|---|---|---|---|
| EC50 (nM) | 1.018 | 0.4284 | 0.4789 |
| Top | 27935 | 26449 | 27811 |
| Bottom | 7898 | 7666 | 8072 |

### Example 4. In vivo anti-tumor activity experiment

The in vivo anti-tumor efficacy of the bispecific antibodies in an animal tumor model were measured in this example.

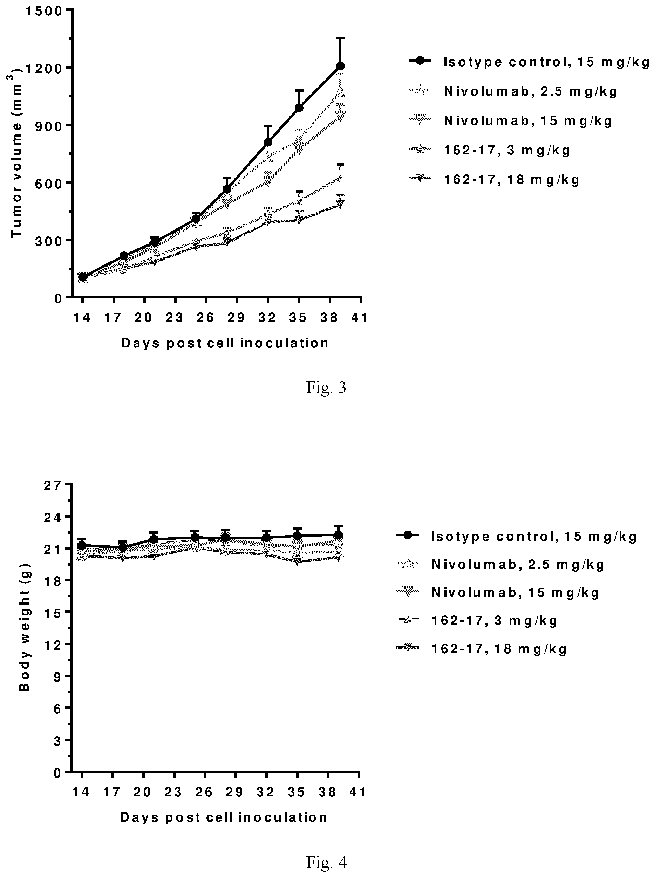

Human breast cancer cells MDA-MB-231 (purchased from Chinese Academy of Sciences) were cultured and expanded in vitro. 5×10⁶ cells were resuspended in PBS and subcutaneously implanted in 6-8 week old female NSG mice (obtained from Shanghai Southern Model Organism Technology Co., Ltd.) to construct a tumor-bearing model. PBMC (obtained from Miaoshun (Shanghai) Biotechnology Co., Ltd.) was injected into the tail vein 7 days after tumor bearing, 4×10⁶ cells/mouse. After 14 days, the mice were divided into groups according to the tumor volume of about 100 mm³ and intraperitoneally injected (twice a week). The body weight and tumor volume were recorded twice a week. The comparative analysis of the tumor volume and body weight of the mice after 39 days was shown in Fig. 3 and Fig. 4, respectively. The results showed that the bispecific antibodies of the present application could significantly inhibit the tumor growth and was significantly better than Nivolumab. In addition, the bispecific antibodies of the present application also had good in vivo safety.

Although the specific embodiments of the present invention have been described in detail, those skilled in the art will understand that various modifications and changes can be made to the details based on all the teachings that have been disclosed, and these changes are within the scope of protection of the present invention. All of the invention is given by the attached claims and any equivalents thereof.

## Claims

1. A multispecific antibody, which comprises a first antigen-binding domain targeting programmed cell death receptor-1 (PD-1) and a second antigen-binding domain targeting vascular endothelial growth factor (VEGF), wherein, the first antigen-binding domain comprises a VHH domain, and the VHH domain comprises: CDR1 as shown in SEQ ID NO: 2 or a variant thereof, CDR2 as shown in SEQ ID NO: 3 or a variant thereof, and CDR3 as shown in SEQ ID NO: 4 or a variant thereof, wherein, the variant has a substitution, deletion or addition of one or several amino acids (e.g., substitution, deletion, or addition of 1, 2, or 3 amino acids) as compared to the sequence from which it is derived;
preferably, the VHH domain comprises: CDR1 as shown in SEQ ID NO: 2, CDR2 as shown in SEQ ID NO: 3, and CDR3 as shown in SEQ ID NO: 4;
preferably, the VHH domain comprises: CDR1, CDR2 and CDR3 as contained in the VHH shown in SEQ ID NO: 1; preferably, the CDR is determined by the Kabat, IMGT, or Chothia numbering systems.

2. The multispecific antibody according to claim 1, wherein the first antigen-binding domain comprises: a VHH comprising the sequence as shown in SEQ ID NO: 1 or a variant thereof, the variant having a sequence identity of at least 80%, at least 85%, at least 90%, at least 91%, at least 92%, at least 93%, at least 94%, at least 95%, at least 96%, at least 97%, at least 98%, at least 99%, or 100% or having a substitution, deletion or addition of one or several amino acids(e.g., substitution, deletion, or addition of 1, 2, 3, 4, 5, 6, 7, 8, 9, or 10 amino acids) as compared to the sequence from which it is derived; preferably, the substitution is a conservative substitution;
preferably, the first antigen-binding domain comprises the VHH as shown in SEQ ID NO: 1.

3. The multispecific antibody according to claim 1 or 2, wherein, the second antigen-binding domain comprises an antibody or antigen-binding fragment thereof (e.g., heavy chain variable region and/or light chain variable region thereof) that specifically binds to VEGF;
preferably, the antibody that specifically binds to VEGF is selected from Bevacizumab or its variant, and Ranibizumab or its variant.

4. The multispecific antibody according to any one of claims 1-3, wherein, the second antigen-binding domain comprises a heavy chain variable region (VH) and a light chain variable region (VL); wherein,
(i) the VH comprises CDR-H1 as shown in SEQ ID NO: 7, CDR-H2 as shown in SEQ ID NO: 8, and CDR-H3 as shown in SEQ ID NO: 9; the VL comprises CDR-L1 as shown in SEQ ID NO: 10, CDR-L2 as shown in SEQ ID NO: 11, and CDR-L3 as shown in SEQ ID NO: 12; or,
(ii) the VH comprises: CDR-H1, CDR-H2, and CDR-H3 as contained in the VH as shown in SEQ ID NO: 5; the VL comprises: CDR-L1, CDR-L2, and CDR-L3 as contained in the VL as shown in SEQ ID NO: 6; preferably, the CDR is determined by the Kabat, IMGT, or Chothia numbering systems;
preferably, the VH and/or VL further comprises a framework region derived from a mammalian (e.g., human or mouse) immunoglobulin;
preferably, the VH comprises the sequence as shown in SEQ ID NO: 5 or a variant thereof, and/or, the VL comprises the sequence as shown in SEQ ID NO: 6 or a variant thereof; the variant having a sequence identity of at least 80%, at least 85%, at least 90%, at least 91%, at least 92%, at least 93%, at least 94%, at least 95%, at least 96%, at least 97%, at least 98%, at least 99%, or 100% or having a substitution, deletion or addition of one or several amino acids (e.g., substitution, deletion, or addition of 1, 2, 3, 4, or 5 amino acids) as compared to the sequence from which it is derived; preferably, the substitution is a conservative substitution;
preferably, the second antigen-binding domain comprises the VH as shown in SEQ ID NO: 5 and the VL as shown in SEQ ID NO: 6.

5. The multispecific antibody according to any one of claims 1-3, wherein, the second antigen-binding domain comprises a heavy chain variable region (VH) and a light chain variable region (VL); wherein,
(i) the VH comprises CDR-H1 as shown in SEQ ID NO: 15, CDR-H2 as shown in SEQ ID NO: 16, and CDR-H3 as shown in SEQ ID NO: 17; the VL comprises CDR-L1 as shown in SEQ ID NO: 18, CDR-L2 as shown in SEQ ID NO: 19, and CDR-L3 as shown in SEQ ID NO: 20; or,
(ii) the VH comprises: CDR-H1, CDR-H2, and CDR-H3 as contained in the VH as shown in SEQ ID NO: 13; the VL comprises: CDR-L1, CDR-L2, and CDR-L3 as contained in the VL as shown in SEQ ID NO: 14; preferably, the CDR is determined by the Kabat, IMGT, or Chothia numbering systems;
preferably, the VH and/or VL further comprises a framework region derived from a mammalian (e.g., human or mouse) immunoglobulin;
preferably, the VH comprises the sequence as shown in SEQ ID NO: 13 or a variant thereof, and/or the VL comprises the sequence as shown in SEQ ID NO: 14 or a variant thereof; the variant having a sequence identity of at least 80%, at least 85%, at least 90%, at least 91%, at least 92%, at least 93%, at least 94%, at least 95%, at least 96%, at least 97%, at least 98%, at least 99%, or 100% or having a substitution, deletion or addition of one or several amino acids (e.g., substitution, deletion, or addition of 1, 2, 3, 4, or 5 amino acids) as compared to the sequence from which it is derived; preferably, the substitution is a conservative substitution;
preferably, the second antigen-binding domain comprises the VH as shown in SEQ ID NO: 13 and the VL as shown in SEQ ID NO: 14.

6. The multispecific antibody according to any one of claims 1-5, wherein, the first antigen-binding domain and the second antigen-binding domain are connected optionally via a linker;
preferably, the linker is a peptide linker, such as a flexible peptide linker;
preferably, the linker is a peptide linker comprising one or more glycines and/or one or more serines, such as a flexible peptide linker comprising (G₄S) n, wherein n is 1, 2, 3, or 4.

7. The multispecific antibody according to any one of claims 1-6, wherein, the second antigen-binding domain is a full-length antibody (e.g., IgG antibody), an Fv fragment, an Fab fragment, an F(ab')₂ fragment, or an scFv.

8. The multispecific antibody according to claim 7, wherein, the second antigen-binding domain comprises a heavy chain and a light chain, the heavy chain comprising a heavy chain variable region and a heavy chain constant region, and the light chain comprising a light chain variable region and a light chain constant region;
preferably, the heavy chain variable region and light chain variable region are as defined in any one of claims 3-5;
preferably, the heavy chain constant region is of IgG, such as IgG1, IgG2, IgG3, or IgG4;
preferably, the heavy chain constant region comprises a sequence as shown in SEQ ID NO: 21;
preferably, the light chain constant region is a κ or λ light chain constant region;
preferably, the light chain constant region comprises a sequence as shown in SEQ ID NO: 22.

9. The multispecific antibody according to claim 8, wherein the first antigen-binding domain is connected to the N-terminus and/or C-terminus of the heavy chain, and/or the N-terminus and/or C-terminus of the light chain optionally via a linker;
preferably, the second antigen-binding domain comprises: at least one heavy chain and at least one light chain, and the first antigen-binding domain is connected to the N-terminus or C-terminus of the heavy chain optionally via a linker, or to the N-terminus of the light chain;
preferably, the second antigen-binding domain comprises: two identical heavy chains and two identical light chains, and the first antigen-binding domain is connected to the N-terminus or C-terminus of both heavy chains optionally via a linker, or to the N-terminus of both light chains.

10. The multispecific antibody according to any one of claims 1-9, comprising:
(1) a first peptide chain comprising a sequence as shown in SEQ ID NO: 23, and a second peptide chain comprising a sequence as shown in SEQ ID NO: 24;
(2) a first peptide chain comprising a sequence as shown in SEQ ID NO: 25, and a second peptide chain comprising a sequence as shown in SEQ ID NO: 26;
(3) a first peptide chain comprising a sequence as shown in SEQ ID NO: 27, and a second peptide chain comprising a sequence as shown in SEQ ID NO: 28;
(4) a first peptide chain comprising a sequence as shown in SEQ ID NO: 29, and a second peptide chain comprising a sequence as shown in SEQ ID NO: 30;
(5) a first peptide chain comprising a sequence as shown in SEQ ID NO: 31, and a second peptide chain comprising a sequence as shown in SEQ ID NO: 32; or,
(6) a first peptide chain comprising a sequence as shown in SEQ ID NO: 33, and a second peptide chain comprising a sequence as shown in SEQ ID NO: 34.

11. The multispecific antibody according to any one of claims 1-10, wherein, the multispecific antibody is a bispecific antibody.

12. The multispecific antibody according to any one of claims 1-10, wherein, the multispecific antibody further comprises at least one additional antigen-binding domain targeting an antigen different from those bound by the first and second antigen-binding domains;
preferably, the multispecific antibody is a trispecific or tetraspecific antibody.

13. An isolated nucleic acid molecule, which comprises a nucleotide sequence encoding the multispecific antibody according to any one of claims 1-12 or at least one polypeptide chain thereof;
preferably, the isolated nucleic acid molecule comprises nucleotide sequences each encoding a polypeptide chain of the multispecific antibody, wherein the nucleotide sequences are present on the same isolated nucleic acid molecule or different isolated nucleic acid molecules.

14. A vector, which comprises the nucleic acid molecule according to claim 13;
preferably, the vector comprises nucleotide sequences each encoding a polypeptide chain of the multispecific antibody according to any one of claims 1-12, wherein the nucleotide sequences are present on the same vector or different vectors.

15. A host cell, which comprises the nucleic acid molecule according to claim 13 or the vector according to claim 14.

16. A method for preparing the multispecific antibody according to any one of claims 1-12, comprising culturing the host cell according to claim 15 under a condition that allows protein expression, and recovering the multispecific antibody from the cultured host cell.

17. A pharmaceutical composition, which comprises the multispecific antibody according to any one of claims 1-12, the isolated nucleic acid molecule according to claim 13, the vector according to claim 14, or the host cell according to claim 15, and a pharmaceutically acceptable carrier and/or excipient;
preferably, the pharmaceutical composition further comprises an additional pharmaceutically active agent, such as an anti-tumor agent.

18. Use of the multispecific antibody according to any one of claims 1-12, the isolated nucleic acid molecule according to claim 13, the vector according to claim 14, the host cell according to claim 15, or the pharmaceutical composition according to claim 17 in the manufacture of a medicament for preventing and/or treating a tumor;
preferably, the tumor is selected from solid tumors;
preferably, the tumor is selected from lung cancer (e.g., non-small cell lung cancer or small cell lung cancer), liver cancer (e.g., hepatocellular carcinoma), kidney cancer (e.g., renal cell carcinoma), gastric cancer, ovarian cancer, cervical cancer, endometrial cancer, breast cancer (e.g., triple-negative breast cancer), colorectal cancer and brain tumors (e.g., glioblastoma);
preferably, the multispecific antibody, the isolated nucleic acid molecule, the vector, the host cell, or the pharmaceutical composition is administered in combination with an additional pharmaceutically active agent (e.g., an anti-tumor agent).

19. A method for preventing and/or treating a tumor, comprising administering an effective amount of the multispecific antibody according to any one of claims 1-12, the isolated nucleic acid molecule according to claim 13, the vector according to claim 14, the host cell according to claim 15, or the pharmaceutical composition according to claim 17 to a subject in need thereof;
preferably, the tumor is selected from solid tumors;
preferably, the tumor is selected from lung cancer (e.g., non-small cell lung cancer or small cell lung cancer), liver cancer (e.g., hepatocellular carcinoma), kidney cancer (e.g., renal cell carcinoma), gastric cancer, ovarian cancer, cervical cancer, endometrial cancer, breast cancer (e.g., triple-negative breast cancer), colorectal cancer and brain tumors (e.g., glioblastoma);
preferably, the subject is a mammal, such as a human;
preferably, the method further comprises administering a second therapeutic agent (e.g., an anti-tumor agent) or therapy (e.g., surgery, chemotherapy, radiotherapy, targeted therapy, immunotherapy, hormone therapy, gene therapy, or palliative therapy) to the subject.
